# FASCICULE DE BREVET EUROPEEN

(11) **EP 1 722 755 B1**
(45) Date de publication et mention de la délivrance du brevet: **29.08.2007**
(21) Numéro de dépôt: 05717391.6
(22) Date de dépôt: 10.01.2005
(51) Int. Cl.: A61K 8/49, A61K 8/896, A61K 8/06, A61K 8/27, A61K 8/29, A61Q 17/04

(54) **COMPOSITION DE TYPE ECRAN SOLAIRE ORGANO-MINERAL ADAPTEE POUR L'APPLICATION PAR POMPE DE PROPULSION**
MINERALORGANISCHE SONNENSCHUTZZUSAMMENSETZUNG ZUM AUFBRINGEN MIT EINER ANTRIEBSPUMPE
ORGANO-MINERAL SUNSCREEN COMPOSITION ADAPTED FOR BEING APPLIED BY A PROPULSION PUMP

(30) Priorité: 09.01.2004 FR 0400174
(43) Date de publication de la demande: 22.11.2006
(73) Titulaire: Laboratoires Expanscience, 92400 Courbevoie (FR)
(72) Inventeur: CHOULOT, Jean-Christophe, F-78120 Rambouillet (FR); PHELOUZAT, Jocelyne, F-28130 Pierres (FR); GUINET, Ludovic, F-92400 Courbevoie (FR)
(74) Mandataire: Warcoin, Jacques
(86) Numéro de dépôt international: PCT/FR2005/000048
(87) Numéro de publication internationale: WO 2005/070381

(56) Documents cités:
- WO-A-03/075875
- DE-A- 10 162 842
- FR-A- 2 817 148

## Description

La présente invention se rapporte à une composition cosmétique ou dermatologique à base d'un mélange d'un agent écran minéral et de l'écran organique ayant pour dénomination commune internationale (DCI) le Méthylène Bis-Benzotriazolyl Tétraméthylbutylphénol, présentant à la fois une très bonne fluidité et une excellente photo-protection à spectre large. Cette composition cosmétique est particulièrement adaptée pour être appliquée sur la peau par une pompe de propulsion.

L'invention concerne ainsi également un dispositif à pompe spécialement conçu pour la composition selon l'invention.

Ce dispositif est particulièrement adapté à la propulsion de ce type de composition, de même que la composition est particulièrement adaptée à ce dispositif. L'ensemble forme donc une combinaison avantageuse.

On sait depuis longtemps que les rayons ultraviolets (UV) émis par le soleil ont non seulement des effets visibles à courts termes (coups de soleil, bronzage) mais provoquent aussi des dégâts à longs termes, en particulier des cancers cutanés. Des écrans solaires adaptés ont été mis au point avec pour cible particulière les enfants, dans la mesure où il a été montré que l'enfance est la période clé en termes de mémorisation des dégâts induits par une photoprotection incomplète. Historiquement, les premiers photoprotecteurs à avoir été utilisés ont été des filtres de synthèse, solubles dans l'huile ou dans l'eau. Ces filtres de synthèse (organiques) ont la propriété d'absorber les rayons UV à la place de la peau mais présentent un certain nombre d'inconvénients, en particulier un indice de protection qui diminue avec le temps et l'ensoleillement (photo-instabilité), une pénétration à travers la peau qui pose la question de leur devenir dans l'organisme (notamment chez l'enfant plus sensible aux effets toxiques) et enfin une absorption des UVB ou UVA mais trop rarement des deux en même temps. Ces dernières années, on a utilisé des agents écrans minéraux (ou "inorganiques") notamment pour des produits solaires pour les enfants. Généralement, il s'agit de dioxyde de titane et d'oxyde de zinc qui ont l'aspect d'une poudre blanche constituée de petites particules de ces pigments. Si ces produits déjà connus présentent certains avantages, en particulier une absence de pénétration à travers la peau, une stabilité au soleil et dans le temps ainsi qu'une totale inertie vis-à-vis des réactions cutanées (irritations, allergies, etc.), il restait jusqu'à présent un inconvénient majeur pour l'utilisateur, à savoir un aspect couvrant très blanc et une difficulté à étaler, empêchant l'obtention d'une protection efficace dans la mesure où soit l'utilisateur ne parvenait pas à appliquer correctement le produit sur toutes les zones à protéger soit il ne cherchait même pas à obtenir une telle application, compte tenu de l'aspect peu esthétique et du caractère peu pratique de ces produits couvrants très blanc et pâteux, d'où une photo-protection incomplète des zones à protéger en priorité, c'est à dire le visage, le cou, les épaules, les mains et les pieds.

En fait, la viscosité importante des produits existants résulte d'un problème de dispersion non homogène des pigments minéraux dans les excipients connus. En effet, une dispersion non homogène empêchant non seulement l'obtention d'une bonne photo-protection (indice de protection élevé) mais aussi d'une photo-protection à spectre large (UVB, UVA courts et UVA longs) même dans les zones où l'application du produit peut sembler correcte à l'utilisateur, il était nécessaire jusqu'à ce jour de compenser ce manque d'homogénéité en augmentant la quantité de pigment minéral, donc la viscosité du produit.

On connaît un lait écran solaire contenant un agent écran inorganique et le même type d'émulsionnant siliconé à composante glucosique par le document WO01/74294 au nom de la demanderesse. Toutefois, les formulations qui y sont décrites ne permettent pas une application par pompe spray ni même par pompe à jet. En effet, la viscosité atteinte par les formulations qui y sont décrites est de l'ordre de 10 fois plus élevée que celle que les compositions de la présente invention permettent d'obtenir. Des exemples comparatifs sont ci-après rassemblés, qui permettent de bien comprendre les améliorations apportées par rapport aux compositions de l'état de l'art.

Le document brevet FR-A-2 817 148 (OREAL) 31 mai 2002 (2002-05-31) décrit une composition cosmétique pour la protection contre les UV (voir revendication 20), de type E/H, comprenant un oxyde métallique (voir revendication 18) et un polydiméthysiloxane oxyalkyléné ayant au moins un reste glucoside (voir revendication 18). L'oxyde métallique est de préférence un nanopigment à concentration de 7-15% (voir page 4, ligne 19 et page 6, ligne 30).

Le document brevet WO 03/075875 A (BORSOS ELEK ; HAASE JUERG (CH); CIBA SC HOLDING AG (CH); EHLIS THOMAS) 18 septembre 2003 (2003-09-18) décrit une pompe à spray (voir page 32, ligne 1), contenant une composition comprenant un benzotriazole (voir revendication 1) et un dérivé siliconé à composante glycoside (voir page 19, dernier paragraphe).

On a maintenant constaté de manière tout à fait surprenante et inattendue que l'association d'un mélange d'un agent écran minéral et de l'écran organique ayant pour dénomination commune internationale (DCI) le Méthylène Bis-Benzotriazolyl Tétraméthylbutylphénol avec certains agents émulsionnants permet d'obtenir une composition cosmétique ou dermatologique présentant à la fois une très bonne fluidité et une excellente photo-protection qui plus est à spectre large tout en conservant une bonne stabilité.

La composition cosmétique ou dermatologique selon l'invention fournit ainsi tout d'abord une protection de surface spectaculaire, se traduisant par des indices de protection pouvant être supérieurs à 30, comme illustré par les exemples ci-après, tout en présentant une très bonne fluidité, c'est à dire une viscosité pouvant être inférieure à 10 Pa.s (10 000 centipoises) à 25°C.

En outre, non seulement cette composition cosmétique ou dermatologique n'a plus l'inconvénient d'un aspect visqueux voire pâteux mais, aussi, elle présente une transparence totale (aucun effet blanchissant), avantage déterminant sur le plan cosmétique pour un produit solaire à haute protection. Elle présente de surcroît l'avantage majeur de pouvoir être adaptée à une application par un dispositif de type flacon à pompe de propulsion (jet ou spray), et ce plus particulièrement grâce à la pompe particulière décrite ci-après fonctionnant par propulsion manuelle.

La présente invention a ainsi pour objet une composition cosmétique ou dermatologique pour la protection contre les rayons ultraviolets, à base d'un mélange d'agents écrans minéraux et de l'écran organique ayant pour dénomination DCI le Méthylène Bis-Benzotriazolyl Tétraméthylbutylphénol, caractérisée en ce qu'elle se présente sous forme d'une émulsion eau-dans-huile, et qu'elle contient au moins un agent émulsionnant choisi dans le groupe constitué par les dérivés siliconés à composante glucosique comprenant un nombre de motifs glucose compris entre 2 et 10, l'agent écran inorganique particulaire étant dispersé de manière homogène dans l'émulsion eau-dans-huile et sa taille moyenne de particule étant comprise entre 1 et 100 nanomètres, et l'agent écran inorganique particulaire étant présent à hauteur de 4 à 40% en poids.

On propose également selon l'invention un ensemble d'application de composition cosmétique ou dermatologique pour la protection contre les rayons ultraviolets, comprenant une telle composition et un contenant de cette composition, ledit contenant étant constitué d'un réservoir et d'une pompe de propulsion par entraînement manuel, caractérisé en ce que la composition se présente sous forme d'une émulsion eau-dans-huile, et qu'elle contient au moins un agent émulsionnant choisi dans le groupe constitué par les dérivés siliconés à composante glucosique comprenant un nombre de motifs glucose compris entre 2 et 10, l'agent écran inorganique particulaire étant dispersé de manière homogène dans l'émulsion eau-dans-huile et sa taille moyenne de particule étant comprise entre 1 et 100 nanomètres, et l'agent écran inorganique particulaire étant présent à hauteur de 4 à 40% en poids.

On propose également selon l'invention un procédé de traitement cosmétique de la peau destiné à protéger la peau contre la nocivité et l'agression des ultra-violets et qui consiste à diffuser par un flacon à pompe de propulsion une quantité efficace d'une composition cosmétique, à l'aide d'un tel ensemble.

On propose également selon l'invention une utilisation de telles compositions pour la fabrication de compositions destinées à être diffusée par un flacon pompe de propulsion sur la peau dans le but de protéger la peau contre la nocivité et l'agression des ultra-violets.

On propose également selon l'invention une utilisation d'un dispositif de type flacon à pompe de propulsion pour l'application sur la peau d'une telle composition.

L'écran organique ayant pour dénomination DCI le Méthylène Bis-Benzotriazolyl Tétraméthylbutylphénol est également connu sous son nom de marque Tinosorb M.

Au sens de la présente invention, on entend par dérivés siliconés à composante glucosique, tous dérivés siliconés comprenant un nombre de motifs glucose compris entre 2 et 10. On préfère, à titre de dérivé siliconé, les (C₂-C₃₀)alkylsilicones et les amino(C₂-C₃₀)alkylsilicones. Ainsi, parmi les dérivés siliconés à composante glucosique selon la présente invention, on peut notamment citer les dérivés obtenus par la réaction des polymères de diméthicone avec des polymères de glucose. Comme polymères de diméthicone, on peut citer à titre d'exemples, l'amino bispropyl diméthicone, l'aminopropyl diméthicone, l'amodiméthicone, la cétyldiméthicone, l'hexyldiméthicone, l'octyl diméthicone et la stéaryl diméthicone.

Dans un mode particulier de réalisation de la composition selon l'invention, le dérivé siliconé à composante glucosique est le produit de la réaction de l'octyldiméthicone avec un polymère de glucose, appelé éthylhexyl diméthicone éthoxy glucoside (dénomination INCI, ethylhexyl dimethicone ethoxy glucoside n° 528 in International Cosmetic Ingredients Dictionary and Handbook, 8ème édition). Un autre nom déposé est le Siliconepolyglucoside.

Selon la présente invention, la composition peut comprendre en outre au moins un autre agent émulsionnant classique, notamment la cyclodiméthicone.

Dans un mode de réalisation particulier de la composition selon l'invention, la proportion d'agent émulsionnant est comprise entre environ 2 et environ 30 % en poids, par rapport au poids total de la composition.

Dans le cadre de la présente invention, on entend par agent écran minéral un écran inorganique particulaire, choisi dans le groupe constitué par le dioxyde de titane, l'oxyde de zinc et les mélanges de ces derniers. Les écrans minéraux peuvent également être enrobés par des ingrédients de natures diverses (acides gras, silicones, métaux, ...).

Parmi les dioxydes de titane utilisés comme agent écran inorganique, on peut citer les dioxydes de titane hydrophiles ou hydrophobes, de préférence les dioxydes de titane dopés en fer. Parmi les dioxydes de titane disponibles dans le commerce, on peut citer comme dioxyde hydrophile, le Titanium dioxide P 25 S (75 % anatase et 25 % rutile), comme dioxyde hydrophobe le Titanium dioxide T 805, comme dioxyde hydrophile dopé en fer le Titanium dioxide PF 2 et comme dioxyde hydrophobe dopé en fer le Titanium dioxide T 817, de la société DEGUSSA. Les oxydes de titane mis en oeuvre dans la présente invention peuvent également être synthétisés selon le procédé AEROSIL® développé par la société DEGUSSA. Ce procédé implique notamment la co-calcination des chlorures de titane et de fer, respectivement TiCl₄ et FeCl₃, à haute température et en présence d'une flamme oxohydrogénée. Les oxydes de titane synthétisés, dopés au fer, présentent une surface BET moyenne de 50m²/g et une taille moyenne de particule de 21 nm.

Aussi, il a été démontré que la capacité d'absorption des UVA et UVB des oxydes de titane augmentait avec leur dispersion. L'introduction de fer au cours du processus de synthèse conduit à la formation d'oxydes mixtes fer-titane, dans lesquels l'oxyde de titane est plus dispersé. Enfin, l'insertion de fer au sein d'une phase oxyde de titane, majoritairement de type anatase, entraîne une modification des propriétés semiconductrices de l'oxyde de titane et donc de ses propriétés photochimiques.

Dans un mode de réalisation particulier de la composition selon l'invention, l'agent écran inorganique particulaire est un mélange de dioxyde de titane enrobé et d'oxyde de zinc.

Selon la présente invention, la phase grasse peut comprendre également d'autres corps gras cosmétiquement ou dermatologiquement acceptables, notamment des huiles animales, végétales ou minérales et leurs analogues, des benzoates d'alcools gras et des triglycérides d'acides gras.

Selon la présente invention, la phase aqueuse comprend de l'eau et des composés hydrophiles cosmétiquement ou dermatologiquement acceptables, parmi lesquels on peut citer la glycérine, et éventuellement des solvants organiques, comme les alcools inférieurs hydrosolubles.

La composition cosmétique ou dermatologique selon l'invention présente une viscosité inférieure à 10 Pa.s (10 000 centipoises) à 25 ° C, mesurée avec un viscosimètre de Brookfield.

Concernant l'altération de l'ADN des cellules de la peau par les rayons UV, son indispensable réparation, qui limitera la pérennisation des dégâts, est contrôlée par la protéine P53 qui ordonne la réparation ou la destruction cellulaire (apoptose). Au cours de l'exposition solaire, la protéine P53 peut devenir inefficace par mutation au niveau du gène d'où prolifération des cellules anormales dont la reconnaissance et le contrôle sont assurés par le système immunitaire (cellules de Langerhans), dernier rempart avant le processus tumoral.

Concernant la photo-immunosuppression, des récentes études ont montré que les rayons UV altèrent les cellules essentielles de l'immunité cutanée, les cellules de Langerhans. Ils les détruisent ou créent des dysfonctionnements en inhibant leur action de reconnaissance et de protection contre les agents étrangers (bactéries, virus, tumeurs, allergènes).

Ces deux agressions en profondeur de la peau, dues à l'action des rayons UV peuvent aboutir à la formation des cancers cutanés.

Or, si la plupart des produits solaires protègent plus ou moins bien contre les rayons UV, en prévenant les coups de soleil, les travaux les plus récents suggèrent que leur efficacité en particulier contre la photo-immunosuppression induite par les rayons UV n'est que partielle et donc une immunosuppression chronique peut se développer en l'absence de coups de soleil visibles et malgré l'application de ces produits solaires.

Certaines études épidémiologiques ont même montré que le risque de cancers cutanés est plus élevé chez les utilisateurs d'écrans solaires, paradoxe qui s'expliquerait par le fait que l'absence de coups de soleil permettraient des expositions plus prolongées et donc favoriseraient les dommages invisibles des rayonnements UV.

Aussi, la composition selon l'invention peut comprendre en outre au moins un agent protecteur contre l'immunosuppression induite par les rayons ultraviolets, choisi dans le groupe constitué par l'Aloe vera (extrait d'Aloe barbadensis), la vitamine E, l'insaponifiable d'huile de soja et les mélanges de ces derniers, dans une proportion comprise avantageusement entre environ 0,05 % et environ 5 % en poids, par rapport au poids total de la composition.

La composition cosmétique ou dermatologique selon l'invention peut comprendre en outre au moins un agent protecteur de l'ADN des cellules de la peau, choisi dans le groupe constitué par les isoflavones et/ou les sels de zinc, dans une proportion avantageusement comprise entre environ 0,01 % et environ 1 % en poids, par rapport au poids total de la composition, ledit sel étant avantageusement le gluconate de zinc.

Les « isoflavones » utilisables selon ce mode particulier de la présente invention sont obtenues par synthèse chimique ou sont des substances naturelles extraites de produits naturels, notamment à partir de végétaux tels que le soja, le trèfle, le lupin, les pépins de pomme etc. Bien souvent les compositions topiques selon la présente invention contiennent, à titre d'isoflavones un mélange de différentes isoflavones, mais elles peuvent également être présentes sous forme pure dans le cadre de la présente invention. Par ailleurs, on distingue les formes aglycones des isoflavones et les formes glycosylées de ces dernières. Ces diverses formes se trouvent le plus souvent en mélange. Elles sont illustrées par les formules suivantes.
Formes aglycones, de formule : 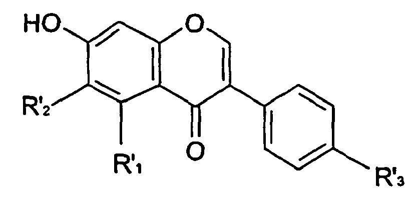 dans laquelle R'₁ représente un atome d'hydrogène ou un groupe hydroxy, R'₂ représente un atome d'hydrogène ou un groupe méthoxy et R'₃ représente un groupe hydroxy.

Avantageusement, selon la présente invention, R'₁, R'₂ et R'₃ représentent :

| **R'₁** | **R'₂** | **R'₃** | **Nom du composé** |
|---|---|---|---|
| H | H | OH | Daidzéine |
| OH | H | OH | Génistéine |
| H | OCH₃ | OH | Glycitéine |

Formes glycosylées, de formule : 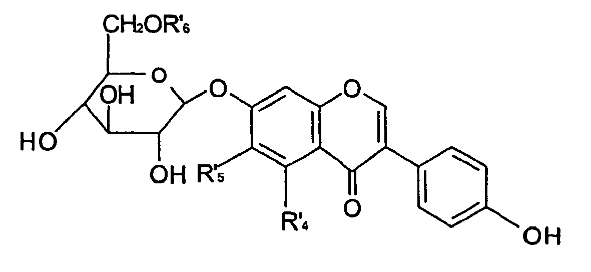 dans laquelle R'₄ représente un atome d'hydrogène ou un groupe hydroxy, R'₅ représente un atome d'hydrogène ou un groupe méthoxy et R'₆ représente un atome d'hydrogène.

Avantageusement, selon la présente invention R'₄, R'₅ et R'₆ représentent :

| **R'₄** | **R'₅** | **R'₆** | **Nom du composé** |
|---|---|---|---|
| H | H | H | Daidzine |
| OH | H | H | Génistine |
| H | OCH₃ | H | Glycitine |

Les formes glycosylées des isoflavones sont les plus abondantes dans la nature.

On préfère, à titre d'isoflavones, les isoflavones naturelles telles que la génistéine (1), la daidzéine ou la glycitéine.

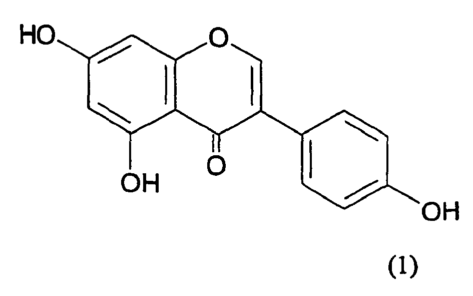

En particulier, la génistéine ou 4,5,7-trihydroxyisoflavone utilisable selon la présente invention peut être un produit d'origine végétale et notamment de soja, titrant 85 à 90 % en poids de génistéine, notamment le produit commercialisé par la société Buckton Scott sous le nom "génistéine titrée à 85%".

Ainsi, la composition cosmétique ou dermatologique selon l'invention présente également, en plus de cette photo-protection à spectre large obtenue par l'association spécifique agent écran minéral - agent émulsionnant, l'avantage d'un effet protecteur "en profondeur" contre l'altération de l'ADN des cellules de la peau et plus particulièrement contre le phénomène de photo-immunosuppression.

Bien entendu, la composition selon l'invention peut également contenir un ou plusieurs adjuvants cosmétiques, lipophiles ou hydrophiles, classiques, notamment ceux qui sont déjà utilisés de manière habituelle dans la fabrication et l'obtention des compositions cosmétiques ou dermatologiques solaires.

Ainsi, la composition cosmétique ou dermatologique selon l'invention peut comprendre en outre au moins un adjuvant choisi dans le groupe constitué par les épaississants ioniques ou non ioniques, les adoucissants, les antioxydants, les opacifiants, les stabilisants, les émollients, les agents répulsifs contre les insectes, les agents hydratants, les vitamines, les parfums, les conservateurs, les charges, les séquestrants, les colorants et les mélanges de ces composés.

La composition cosmétique ou dermatologique selon l'invention peut être préparée par toute méthode connue de l'homme du métier, notamment par mélange des différents ingrédients.

La composition cosmétique ou dermatologique selon l'invention peut se présenter sous forme d'une crème, d'un lait, d'un gel, d'un gel-crème, ou sous toutes autres formes généralement adaptées pour le conditionnement en pompe spray ou en pompe à jet et destinées à être utilisées en cosmétique ou dermatologie pour l'application topique d'une émulsion eau-dans-huile, en particulier celles convenant habituellement aux compositions cosmétiques ou dermatologiques solaires.

Un autre objet de la présente invention réside dans un procédé de traitement cosmétique de la peau destiné à protéger la peau contre la nocivité et l'agression des ultra-violets et qui consiste à diffuser par une pompe spray ou jet sur celle-ci une quantité efficace d'une composition cosmétique telle que définie ci-dessus.

Les compositions selon l'invention peuvent donc être utilisées dans le traitement des pathologies liées aux UVA et aux WB, notamment les érythèmes, l'acné, le vieillissement, l'immunosuppression, l'inflammation ainsi que l'aggravation d'autres pathologies dermatologiques (acné, rosacée, ...).

Le dispositif de type à pompe de propulsion qui est spécialement préconisé ici, et qui s'avère avantageuse tout spécifiquement pour la présente composition est le suivant.

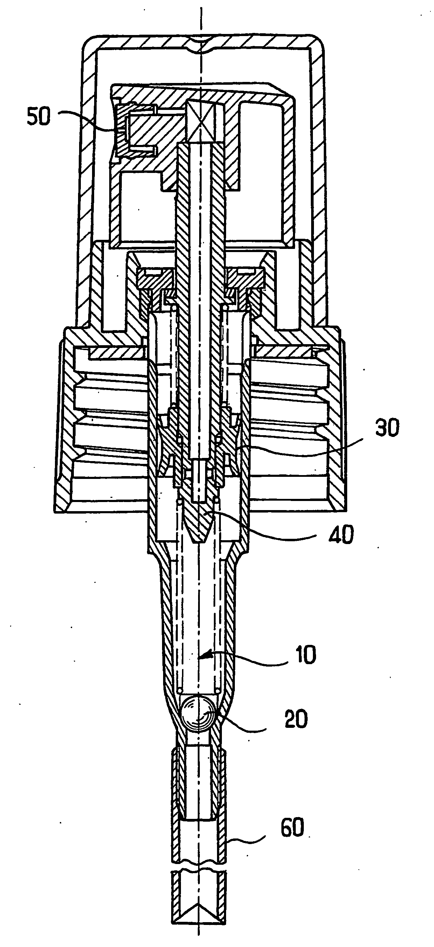

Cette pompe est illustrée plus spécifiquement à la figure unique qui est annexée à la présente description. La présente pompe est une pompe à jet, c'est-à-dire délivrant un flux de ladite composition solaire sous une forme relativement ciblée.

Toutefois une utilisation de la pompe en déplacement lors de cette propulsion permet un certain étalement du produit sur la peau et notamment la répartition voulue.

Cette pompe est conçue autour de l'utilisation d'une chambre intermédiaire 10 de forme sensiblement cylindrique et coaxiale avec l'ensemble du flacon (réservoir non représenté).

Cette chambre intermédiaire est délimitée à ses extrémités supérieures et inférieures par des dispositifs antiretour respectifs. Le dispositif antiretour inférieur, c'est-à-dire adjacent au réservoir, est composé d'une bille 20 reposant sur un col en entonnoir lorsque rappelée dans cette position par un ressort associé.

Le dispositif antiretour supérieur fonctionne lui aussi sur le principe d'un élément obstruant déplaçable verticalement dans la chambre. L'élément obstruant est ici un piston 30, sous forme de joint annulaire, entourant un gicleur 40, ici sous forme d'un pointeau mobile destiné à envahir le volume de la chambre lors d'une pression descendante par l'utilisateur, le gicleur étant alors apte à véhiculer la composition en direction de la buse.

Le principe de cet élément antiretour 30 repose sur le fait que le gicleur 40, coulissé verticalement lors de l'actionnement de la pompe, voit ses ouvertures d'extrémité alors dégagées par ce piston circulaire 30 afin de permettre à la composition, reposant précédemment dans la chambre 10, de s'introduire à l'intérieur du gicleur 40. Le piston 30 se trouve alors légèrement décalé par rapport à ces ouvertures.

Au contraire, lorsque le gicleur 40 est relâché par l'utilisateur, c'est à dire rappelé vers le haut sous la pression d'un ressort hélicoïdal, le piston 30 tend par friction à reprendre sa position devant les ouvertures de circulation internes du gicleur 40, rendant celui-ci alors étanche et, sous le déplacement de ce dernier vers le haut, le piston 30 assure alors la succion de la composition depuis le réservoir vers l'intérieur de la chambre intermédiaire 10.

Aussi, lorsque le gicleur 40 retrouve sa position de repos en extension pour la partie supérieure de la pompe, le piston 30 se trouve également en position d'obstruction, empêchant toute fuite de composition hors du gicleur 40 et hors de la pompe.

Autrement dit, et pour résumer le fonctionnement de cette pompe, l'élément antiretour supérieur 30 libère des ouvertures de circulation d'un élément mécanique venant envahir l'intérieur de la chambre intermédiaire 10 chargée en composition, autorisant la montée de la composition à l'intérieur de cet élément 40. Au contraire, cet élément antiretour 30 empêche la sortie de la composition hors du gicleur et vers la chambre intermédiaire lorsque le gicleur se déplace vers le haut, emportant ainsi la composition avec lui.

Il s'agit donc, pour l'élément anti-retour supérieur, d'un élément associé à un organe mobile de puisage de la composition.

La présente pompe est montée sur un moyen de vissage destiné à un réservoir relativement classique.

Toutefois, pour la propulsion de la composition ci-dessus et ci-après décrite en détail, cette pompe a été dimensionnée pour fournir un jet particulièrement efficace, et pour empêcher des dysfonctionnements ultérieurs d'une telle pompe par exemple par colmatage ou refoulage, cesdits phénomènes correspondant à une mauvaise circulation à l'intérieur de celle-ci

Pour cette efficacité de propulsion, la pompe présente des dimensions particulières dans différentes zones de circulation de la composition.

Ainsi, en parcourant le flux de la composition depuis le réservoir jusqu'à la buse de sortie, les éléments suivants ont été adoptés, et se sont avérés particulièrement adaptés à la présente composition et plus généralement à ce type de fluidité dans le cadre des compositions solaires.

Ainsi un tube plongeur 60 s'étendant dans le réservoir présente un diamètre intérieur majoré ici à 3,70 mm, particulièrement adapté pour des crèmes de moins de 10 Pa.s (10 000 centipoises) à 25°C.

La bille 20 présente, elle, un diamètre de 3 mm, et le corps de pompe présente un diamètre extérieur de 8,6 mm, la chambre intermédiaire correspondante 10 ayant donc un diamètre inférieur, sensiblement égal à 8 mm.

Ces derniers aménagements sont plus particulièrement favorables à la suppression des risques de colmatage de crème à ces endroits au cours des délivrances successives.

Le gicleur 40 est rappelé vers le haut par un ressort hélicoïdal, précédemment décrit, dont la force de compression est comprise entre 35 et 40 newtons, pour une puissance de succion suffisante. Le piston 30 est, quant à lui, rappelé dans sa position d'obstruction, lui-même par un ressort hélicoïdal dont la force est comprise entre 35 et 40 newtons.

On notera que la force de rappel totale est préférentiellement supérieure à 35 newtons, pour une succion suffisante de la composition en présence.

Plus spécifiquement, le ressort inférieur est ici dimensionné à 40 newtons et le ressort associé au piston 30 et rappelant également le gicleur 40 vers le haut via un col d'appui associé, présente quant à lui une force de 35 newtons.

Au total, cette pompe présente un volume de chambre intermédiaire 10 qui est très avantageusement compris entre 100 et 200 µl et encore plus préférentiellement entre 150 et 200 µl.

Enfin la buse 50 présente un diamètre compris entre 4 et 5 mm permettant la vidange de la chambre intermédiaire 10 sans générer de phénomène de refoulage, c'est-à-dire sans naissance de flux parasites en direction opposée à celui de la sortie.

Cette buse 50, outre la préservation d'un bon fonctionnement de la pompe au cours de la durée de vie de cette dernière s'avère permettre la délivrance d'une dose de crème nécessaire et suffisante pour une bonne protection solaire et pour l'application suffisante de la présente composition sur la peau.

La précision du jet unidirectionnel condensé qui est obtenu permet de délivrer sur une zone souhaitée une grande quantité de cette composition, favorisant une protection solaire optimale.

Bien que la présente pompe vise la délivrance d'un jet, elle est également adaptée, sous réserve de quelques aménagements facilement accessibles grâce aux connaissances habituelles de l'homme du métier, à la délivrance d'un spray. Un spray, ou pulvérisation, vise lui l'application sur une zone plus étendue.

Les exemples qui suivent illustrent l'invention sans toutefois la limiter à ces modes particuliers de réalisation.

Sauf indications contraires, les pourcentages indiqués dans les exemples qui suivent sont des pourcentages en poids total de la composition.

### Exemple 1 : Mode opératoire pour produits solaires

### 1- PREPARATION DE LA PHASE GRASSE

Dans un réacteur, on introduit la phase grasse (pigments minéraux, émulsionnant et huile) et on homogénéise avec recyclage pour obtenir une bonne dispersion.

On chauffe cette phase jusqu'à 60°C.

### 2 - PREPARATION DE LA PHASE AQUEUSE

Dans un conge, sous agitation, on disperse le gélifiant dans la phase aqueuse contenant les électrolytes, puis on ajoute les actifs.

On chauffe cette phase jusqu'à 60°C. Puis on introduit le Méthylène Bis-Benzotriazolyl Tétraméthylbutylphénol.

### 3 - MISE EN EMULSION

A 60°C, on coule la phase aqueuse lentement (30 min) dans la phase huileuse, en maintenant une agitation assez soutenue (à l'aide d'une turbine ou d'un racleur) et on vérifie l'aspect de l'émulsion.

On homogénéise pendant 30 min sous recyclage en refroidissant le produit jusqu'à 25°C.

On vidange, puis on contrôle le produit après 1 heure de repos pour obtenir une indication de sa viscosité. On vérifie la parfaite régularité de l'émulsion.

### 4 - MESURE DE LA VISCOSITE

La viscosité est mesurée à 25°C avec un viscosimètre de Brookfiled.

| Emulsionnant | IP | Viscosité à 25 °C Pa.s (cp) |
|---|---|---|
| ETA (Cétyldiméthicone polyols) | 35 | 200 (200 000) |
| ETA (Cétyldiméthicone polyols) | 20 | 60 (60 000) |
| ETA (exemple 1) | 25 | 15 (15 000) |
| Invention (exemple 2) | 32 | 7 (7 000) |
| Invention (exemple 3) | 40 | 10 (10 000) |

Ainsi pour un indice de protection (IP) voisin, les compositions selon l'invention possèdent une viscosité nettement inférieure à celles des compositions de l'état antérieur de la technique (ETA).

### Exempte 1 : Formule avec un indice de protection supérieur à 25 (exemple comparatif)

| **Ingrédients** | **% en poids** |
|---|---|
| Eau | 30 à 60 |
| Tetraoctanoate de Pentaerythrityle | 15 à 30 |
| Dioxyde de titane | 1 à 10 |
| Cyclométhicone | 1 à 10 |
| Oxyde de zinc | 1 à 10 |
| Benzoate de (C12-C15)alkyle | 1 à 10 |
| 2-Heptadecadiènylfurane | 0,1 à 10 |
| 4,5,7-Trihydroxyisoflavone | 0,01 à 10 |
| Glycérine | 1 à 10 |
| Ether de dicaprylyle | 1 à 10 |
| Cyclopentasiloxane | 1 à 10 |
| Ethylhexyl dimethicone Ethoxy Glucoside | 1 à 10 |
| Propylene Glycol Dioctanoate | 1 à 10 |
| Chlorure de sodium | 1 à 5 |
| PEG-45/Dodecyl Glycol Copolymer | 1 à 5 |
| PEG-30 Dipolyhydroxystéarate | 1 à 5 |
| Huile d'insaponifiable de soja | 1 à 5 |
| Palmitate de dextrine | 1 à 5 |
| Phénoxyéthanol | 0,5 |
| Extrait *d'aloe barbadensis* | 0,2 |
| Méthylparaben | 0,16 |
| Gluconate de zinc | 0,08 |
| Butylparaben | 0,06 |
| Ethylparaben | 0,04 |
| Propylparaben | 0,02 |

### Exemple 2 : Formule avec un indice de protection supérieur à 30

| **Ingrédients** | **% en poids** |
|---|---|
| Eau | 20 à 50 |
| Tetraoctanoate de Pentaerythrityle | 15 à 30 |
| Dioxyde de titane | 1 à 10 |
| Cyclométhicone | 1 à 10 |
| Oxyde de zinc | 1 à 10 |
| Methylene Bis-Benzotriazolyl Tetramethylbutylphenol | 1 à 5 |
| Benzoate de (C12-C15)alkyle | 1 à 10 |
| 2-Heptadecadiènylfurane | 0,1 à 10 |
| 4,5,7-Trihydroxyisoflavone | 0,01 à 10 |
| Glycérine | 1 à 10 |
| Ether de dicaprylyle | 1 à 10 |
| Cyclopentasiloxane | 1 à 10 |
| Ethylhexyl dimethicone Ethoxy Glucoside | 1 à 10 |
| Propylene Glycol Dioctanoate | 1 à 10 |
| Chlorure de sodium | 1 à 5 |
| PEG-45/Dodecyl Glycol Copolymer | 1 à 5 |
| PEG-30 Dipolyhydroxystéarate | 1 à 5 |
| Huile d'insaponifiable de soja | 1 à 5 |
| Palmitate de dextrine | 1 à 5 |
| Phénoxyéthanol | 0,5 |
| Extrait *d'aloe barbadensis* | 0,2 |
| Méthylparaben | 0,16 |
| Gluconate de zinc | 0,08 |
| Butylparaben | 0,06 |
| Ethylparaben | 0,04 |
| Propylparaben | 0,02 |

### Exemples 3 : Formule avec un indice de protection supérieur à 40

| **Ingrédients** | **% en poids** |
|---|---|
| Eau | 20 à 50 |
| Tetraoctanoate de Pentaerythrityle | 15 à 30 |
| Dioxyde de titane | 1 à 10 |
| Cyclométhicone | 1 à 10 |
| Oxyde de zinc | 1 à 10 |
| Methylene Bis-Benzotriazolyl Tetramethylbutylphenol | 1 à 10 |
| Benzoate de (C12-C15)alkyle | 1 à 10 |
| 2-Heptadecadiènylfurane | 0,1 à 10 |
| 4,5,7-Trihydroxyisoflavone | 0,01 à 10 |
| Glycérine | 1 à 10 |
| Ether de dicaprylyle | 1 à 10 |
| Cyclopentasiloxane | 1 à 10 |
| Ethylhexyl dimethicone Ethoxy Glucoside | 1 à 10 |
| Propylene Glycol Dioctanoate | 1 à 10 |
| Chlorure de sodium | 1 à 5 |
| PEG-45/Dodecyl Glycol Copolymer | 1 à 5 |
| PEG-30 Dipolyhydroxystéarate | 1 à 5 |
| Huile d'insaponifiable de soja | 1 à 5 |
| Palmitate de dextrine | 1 à 5 |
| Phénoxyéthanol | 0,5 |
| Extrait *d'aloe barbadensis* | 0,2 |
| Méthylparaben | 0,16 |
| Gluconate de zinc | 0,08 |
| Butylparaben | 0,06 |
| Ethylparaben | 0,04 |
| Propylparaben | 0,02 |

### Exemple 4 : Méthode de détermination du facteur de protection solaire

Le niveau de protection solaire fait intervenir la réponse érythémale de la peau aux radiations ultraviolettes. Elle est exprimée par le facteur de protection solaire (FPS) qui est le rapport des énergies nécessaires pour induire une réponse érythémale minimale sur la peau de sujets volontaires protégée ou non par le produit à tester, à l'aide du rayonnement ultra-violet généralement fourni par une source artificielle.

La méthode utilisée est celle du COLIPA (European Cosmetic Toilettery And Perfumes Association) décrite dans « La méthode de détermination du facteur de protection solaire », Réf. : 94/289, octobre 1994).

On détermine chez chaque volontaire la plus faible dose qui produit un érythème, appelée dose érythémale minimale (DEM), soit sans protection (DEMn), soit avec protection (DEMp) et on calcule FPS comme étant le rapport DEMp/DEMn.

Les compositions selon l'invention ont des indices de protection pouvant atteindre 40.

## Revendications

1. Composition cosmétique ou dermatologique pour la protection contre les rayons ultraviolets, à base d'un mélange d'un agent écran minéral et de l'écran organique ayant pour dénomination DCI le Méthylène Bis-Benzotriazolyl Tétraméthylbutylphénol, **caractérisée en ce qu'**elle se présente sous forme d'une émulsion eau-dans-huile, et qu'elle contient au moins un agent émulsionnant choisi dans le groupe constitué par les dérivés siliconés à composante glucosique comprenant un nombre de motifs glucose compris entre 2 et 10, l'agent écran inorganique particulaire étant dispersé de manière homogène dans l'émulsion eau-dans-huile et sa taille moyenne de particule étant comprise entre 1 et 100 nanomètres, et l'agent écran inorganique particulaire étant présent à hauteur de 4 à 40% en poids.

2. Composition cosmétique ou dermatologique selon la revendication 1 **caractérisée en ce que** le dérivé siliconé est choisi parmi les (C₂-C₃₀)alkylsilicones et les amino(C₂-C₃₀)alkylsilicones.

3. Composition cosmétique ou dermatologique selon la revendication 2, **caractérisée en ce que** le dérivé siliconé à composante glucosique est l'éthylhexyl diméthicone éthoxy glucoside ou siliconepolyglucoside.

4. Composition cosmétique ou dermatologique selon l'une quelconque des revendication précédentes, **caractérisée en ce que** la composition cosmétique comprend en outre de la cyclodiméthicone.

5. Composition cosmétique ou dermatologique selon l'une quelconque des revendications précédentes **caractérisée en ce que** la proportion de l'agent émulsionnant est comprise entre environ 2 et environ 30 % en poids, par rapport au poids total de la composition.

6. Composition cosmétique ou dermatologique selon l'une quelconque des revendications précédentes, **caractérisée en ce que** l'agent écran minéral est un écran inorganique particulaire, choisi dans le groupe constitué par le dioxyde de titane, l'oxyde de zinc et les mélanges de ces derniers.

7. Composition cosmétique ou dermatologique selon l'une quelconque des revendications précédentes, **caractérisée en ce que** l'agent écran inorganique particulaire est du dioxyde de titane hydrophobe pouvant être enrobé.

8. Composition cosmétique ou dermatologique selon l'une quelconque des revendications précédentes, **caractérisée en ce qu'**elle présente une viscosité inférieure à 10 Pa.s (10 000 centipoises) à 25 ° C.

9. Composition cosmétique ou dermatologique selon l'une quelconque des revendications précédentes, **caractérisée en ce qu'**elle comprend en outre au moins un agent protecteur contre l'immuno-suppression induite par les rayons ultraviolets, choisi dans le groupe constitué par l'Aloe vera, la vitamine E, l'insaponifiable d'huile de soja et les mélanges de ces derniers.

10. Composition cosmétique ou dermatologique selon la revendication 9, **caractérisée en ce que** la proportion de l'agent protecteur contre l'immuno-suppression induite par les rayons ultraviolets est comprise entre environ 0,05 et environ 5 % en poids, par rapport au poids total de la composition.

11. Composition cosmétique ou dermatologique selon l'une quelconque des revendications précédentes, **caractérisée en ce qu'**elle comprend en outre au moins un agent protecteur de l'ADN des cellules de la peau, choisi dans le groupe constitué par les isoflavones et/ou les sels de zinc.

12. Composition cosmétique ou dermatologique selon la revendication 11, **caractérisée en ce que** l'agent protecteur des cellules de la peau est le gluconate de zinc.

13. Composition cosmétique ou dermatologique selon la revendication 11 ou 12, **caractérisée en ce que** la proportion de l'agent protecteur des cellules de la peau est comprise entre environ 0,01 et environ 1 % en poids, par rapport au poids total de la composition.

14. Composition cosmétique ou dermatologique selon l'une quelconque des revendications précédentes, **caractérisée en ce qu'**elle comprend en outre au moins un adjuvant choisi dans le groupe constitué par les épaississants ioniques ou non ioniques, les adoucissants, les antioxydants, les opacifiants, les stabilisants, les émollients, les agents répulsifs contre les insectes, les agents hydratants, les vitamines, les parfums, les conservateurs, les charges, les séquestrants, les colorants et les mélanges de ces derniers.

15. Ensemble d'application de composition cosmétique ou dermatologique pour la protection contre les rayons ultraviolets, comprenant une telle composition et un contenant de cette composition, ledit contenant étant constitué d'un réservoir et d'une pompe de propulsion (10, 20, 30) par entraînement manuel, **caractérisé en ce que** la composition se présente sous forme d'une émulsion eau-dans-huile, et qu'elle contient au moins un agent émulsionnant choisi dans le groupe constitué par les dérivés siliconés à composante glucosique comprenant un nombre de motifs glucose compris entre 2 et 10, l'agent écran inorganique particulaire étant dispersé de manière homogène dans l'émulsion eau-dans-huile et sa taille moyenne de particule étant comprise entre 1 et 100 nanomètres, et l'agent écran inorganique particulaire étant présent à hauteur de 4 à 40% en poids.

16. Ensemble selon la revendication 15, **caractérisé en ce que** la pompe est une pompe à chambre intermédiaire (16) est à élément coulissant (40) apte à envahir le volume interne de la chambre intermédiaire (10) pour puiser la composition présente dans cette chambre intermédiaire (10).

17. Ensemble selon la revendication 15 ou la revendication 16, **caractérisé en ce que** la pompe (10, 20, 30) est une pompe à chambre intermédiaire (10) encadrée par deux dispositifs anti-retour (20, 30) orientés dans le sens de sortie du réservoir, et **en ce que** l'un des dispositifs anti-retour (20, 30), situé adjacent au réservoir, est une bille d'obstruction (20).

18. Ensemble selon l'une quelconque des revendications 15 à 17, **caractérisé en ce que** la pompe (10, 20, 30) est munie d'une buse de sortie (50) dont l'espace de circulation de la composition présente un diamètre compris entre 4 et 5 mm.

19. Ensemble selon l'une quelconque des revendications 15 à 18, **caractérisé en ce que** la pompe (10, 20, 30) présente une chambre intermédiaire (10) ayant un volume compris entre 100 et 200 µl.

20. Ensemble selon l'une quelconque des revendications 15 à 19, **caractérisé en ce que** la pompe présente un ou plusieurs éléments de rappels assurant le remplissage du volume intermédiaire, produisant au total une force supérieure à 35 N.

21. Ensemble selon l'une quelconque des revendications 15 à 20, **caractérisé en ce que** la pompe présente une chambre intermédiaire de type cylindrique, dont le diamètre est d'environ 8mm.

22. Ensemble selon l'une quelconque des revendications 15 à 21, **caractérisé en ce que** la pompe est munie d'un tube plongeur dont le diamètre interne est d'environ 3,7 mm.

23. Utilisation des compositions selon l'une quelconque des revendications 1 à 14 pour la fabrication de compositions dermatologiques destinées à être diffusées par un flacon à pompe de propulsion sur la peau dans le but de protéger la peau contre la nocivité et l'agression des ultra-violets.

24. Procédé non thérapeutique de traitement cosmétique de la peau destiné à protéger la peau contre la nocivité et l'agression des ultra-violets et qui consiste à diffuser par un flacon à pompe de propulsion une quantité efficace de la composition, à l'aide d'un ensemble conforme à l'une quelconque des revendications 15 à 22.

25. Utilisation d'un dispositif de type flacon à pompe de propulsion pour l'application sur la peau d'une composition selon l'une quelconque des revendications 1 à 14.

## Claims

1. Cosmetic or dermatological composition for protecting against ultraviolet rays, based on a mixture of mineral screening agents and the organic screening agent having the INN name methylenebis(benzotriazolyl)tetramethylbutylphenol, **characterized in that** it is in the form of a water-in-oil emulsion and **in that** it contains at least one emulsifier chosen from the group consisting of silicone derivatives with a glucose component comprising between 2 and 10 glucose units, the particulate inorganic screening agent being uniformly dispersed in the water-in-oil emulsion and its mean particle size being between 1 and 100 nanometers, and the particulate inorganic screening agent being present in a proportion of from 4% to 40% by weight.

2. Cosmetic or dermatological composition according to Claim 1, **characterized in that** the silicone derivative is chosen from (C₂-C₃₀)alkylsilicones and amino (C₂-C₃₀) alkylsilicones.

3. Cosmetic or dermatological composition according to Claim 2, **characterized in that** the silicone derivative with a glucose component is ethylhexyl dimethicone ethoxy glucoside or silicone polyglucoside.

4. Cosmetic or dermatological composition according to any one of the preceding claims, **characterized in that** the cosmetic composition also comprises cyclodimethicone.

5. Cosmetic or dermatological composition according to any one of the preceding claims, **characterized in that** the proportion of emulsifier is between about 2% and about 30% by weight relative to the total weight of the composition.

6. Cosmetic or dermatological composition according to any one of the preceding claims, **characterized in that** the mineral screening agent is a particulate inorganic screening agent chosen from the group consisting of titanium dioxide, zinc oxide, and mixtures thereof.

7. Cosmetic or dermatological composition according to any one of the preceding claims, **characterized in that** the particulate inorganic screening agent is hydrophobic titanium dioxide, which may be coated.

8. Cosmetic or dermatological composition according to any one of the preceding claims, **characterized in that** it has a viscosity of less than 10 Pa.s (10 000 centipoises) at 25°C.

9. Cosmetic or dermatological composition according to any one of the preceding claims, **characterized in that** it also comprises at least one agent for protecting against the immunosuppression induced by ultraviolet rays, chosen from the group consisting of *Aloe vera,* vitamin E, the unsaponifiable matter of soybean oil, and mixtures thereof.

10. Cosmetic or dermatological composition according to Claim 9, **characterized in that** the proportion of agent for protecting against the immunosuppression induced by ultraviolet rays is between about 0.05% and about 5% by weight relative to the total weight of the composition.

11. Cosmetic or dermatological composition according to any one of the preceding claims, **characterized in that** it also comprises at least one agent for protecting the DNA of skin cells, chosen from the group consisting of isoflavones and/or zinc salts.

12. Cosmetic or dermatological composition according to Claim 11, **characterized in that** the agent for protecting skin cells is zinc gluconate.

13. Cosmetic or dermatological composition according to Claim 11 or 12, **characterized in that** the proportion of agent for protecting skin cells is between about 0.01% and about 1% by weight relative to the total weight of the composition.

14. Cosmetic or dermatological composition according to any one of the preceding claims, **characterized in that** it also comprises at least one adjuvant chosen from the group consisting of ionic or nonionic thickeners, softeners, antioxidants, opacifiers, stabilizers, emollients, insect repellents, moisturizers, vitamins, fragrances, preserving agents, fillers, sequestrants, dyes, and mixtures thereof.

15. Assembly for applying a cosmetic or dermatological composition for protecting against ultraviolet rays, comprising such a composition and a container for this composition, said container consisting of a reservoir and a manually-driven propulsion pump (10, 20, 30), **characterized in that** the composition is in the form of a water-in-oil emulsion and **in that** it contains at least one emulsifier chosen from the group consisting of silicone derivatives with a glucose component comprising between 2 and 10 glucose units, the particulate inorganic screening agent being uniformly dispersed in the water-in-oil emulsion and its mean particle size being between 1 and 100 nanometers, and the particulate inorganic screening agent being present in a proportion of from 4% to 40% by weight.

16. Assembly according to Claim 15, **characterized in that** the pump is a pump with an intermediate chamber (16) and a sliding member (40) capable of occupying the inner volume of the intermediate chamber (10) to deplete the composition present in this intermediate chamber (10).

17. Assembly according to Claim 15 or Claim 16, **characterized in that** the pump (10, 20, 30) is a pump with an intermediate chamber (10) surrounded by two nonreturn devices (20, 30) oriented in the direction of outlet of the reservoir, and **in that** one of the nonreturn devices (20, 30), located adjacent to the reservoir, is an obstruction bead (20).

18. Assembly according to any one of Claims 15 to 17, **characterized in that** the pump (10, 20, 30) is equipped with an outlet nozzle (50), the composition circulation space of which has a diameter of between 4 and 5 mm.

19. Assembly according to any one of Claims 15 to 18, **characterized in that** the pump (10, 20, 30) has an intermediate chamber (10) with a volume of between 100 and 200 µl.

20. Assembly according to any one of Claims 15 to 19, **characterized in that** the pump has one or more return members for filling the intermediate volume, producing in total a force of greater than 35 N.

21. Assembly according to any one of Claims 15 to 20, **characterized in that** the pump has an intermediate chamber of cylindrical type, the diameter of which is about 8 mm.

22. Assembly according to any one of Claims 15 to 21, **characterized in that** the pump is equipped with a dip tube with an inside diameter of about 3.7 mm.

23. Use of the compositions according to any one of Claims 1 to 14 for the manufacture of dermatological compositions intended to be diffused via a propulsion pump bottle onto the skin for the purpose of protecting the skin against the harmfulness of and attack by ultraviolet rays.

24. Nontherapeutic cosmetic skin treatment process for protecting the skin against the harmfulness of and attack by ultraviolet rays and which consists in diffusing from a propulsion pump bottle an effective amount of the composition, using an assembly in accordance with any one of Claims 15 to 22.

25. Use of a device of propulsion pump bottle type for applying a composition according to any one of Claims 1 to 14 to the skin.

## Patentansprüche

1. Kosmetische oder dermatologische Zusammensetzung für den Schutz gegen die ultravioletten Strahlen auf der Grundlage einer Mischung eines mineralischen Sonnenschutzmittels und des organischen Sonnenschutzmittels mit Methylenbisbenzotriazolyltetramethylbutylphenol als internationalem freiem Warennamen, **dadurch gekennzeichnet, dass** sie in Form einer Wasser-in-ÖI-Emulsion vorliegt und dass sie wenigstens einen Emulgator, ausgewählt in der Gruppe bestehend aus den Siliconderivaten mit Glucose-Komponente, umfassend eine Anzahl von Glucose-Motiven zwischen 2 und 10 eingeschlossen, enthält, wobei das teilchenförmige anorganische Sonnenschutzmittel auf homogene Weise in der Wasser-in-ÖI-Emulsion dispergiert ist und seine mittlere Teilchengröße zwischen 1 und 100 Nanometern eingeschlossen beträgt und wobei das teilchenförmige anorganische Sonnenschutzmittel in einer Konzentration von 4 bis 40 Gew.-% vorhanden ist.

2. Kosmetische oder dermatologische Zusammensetzung nach Anspruch 1, **dadurch gekennzeichnet, dass** das Siliconderivat unter den (C₂-C₃₀)-Alkylsiliconen und den Amino-(C₂-C₃₀)-alkylsiliconen ausgewählt wird.

3. Kosmetische oder dermatologische Zusammensetzung nach Anspruch 2, **dadurch gekennzeichnet, dass** das Siliconderivat mit Glucose-Komponente Ethylhexyldimethiconethoxyglucosid oder Siliconpolyglucosid ist.

4. Kosmetische oder dermatologische Zusammensetzung nach einem der vorangegangenen Ansprüche, **dadurch gekennzeichnet, dass** die kosmetische Zusammensetzung außerdem Cyclodimethicon umfasst.

5. Kosmetische oder dermatologische Zusammensetzung nach einem der vorangegangenen Ansprüche, **dadurch gekennzeichnet, dass** der Anteil des Emulgators zwischen etwa 2 und etwa 30 Gew.-% eingeschlossen bezogen auf das Gesamtgewicht der Zusammensetzung liegt.

6. Kosmetische oder dermatologische Zusammensetzung nach einem der vorangegangenen Ansprüche, **dadurch gekennzeichnet, dass** das mineralische Sonnenschutzmittel ein teilchenförmiges anorganisches Sonnenschutzmittel ist, welches in der aus Titandioxid, Zinkoxid und den Mischungen von diesen Letzteren bestehenden Gruppe ausgewählt wird.

7. Kosmetische oder dermatologische Zusammensetzung nach einem der vorangegangenen Ansprüche, **dadurch gekennzeichnet, dass** das teilchenförmige anorganische Sonnenschutzmittel hydrophobes Titandioxid, das umhüllt sein kann, ist.

8. Kosmetische oder dermatologische Zusammensetzung nach einem der vorangegangenen Ansprüche, **dadurch gekennzeichnet, dass** sie eine Viskosität unter 10 Pa.s (10000 Centipoise) bei 25°C aufweist.

9. Kosmetische oder dermatologische Zusammensetzung nach einem der vorangegangenen Ansprüche, **dadurch gekennzeichnet, dass** sie außerdem wenigstens ein Schutzmittel gegen die durch die ultravioletten Strahlen induzierte Immunsuppression, welches in der aus Aloe vera, Vitamin E, dem unverseifbaren Anteil von Sojaöl und den Mischungen von diesen Letzteren bestehenden Gruppe ausgewählt wird, umfasst.

10. Kosmetische oder dermatologische Zusammensetzung nach Anspruch 9, **dadurch gekennzeichnet, dass** der Anteil des Schutzmittels gegen die durch die ultravioletten Strahlen induzierte Immunsuppression zwischen etwa 0,05 und etwa 5 Gew.-% eingeschlossen bezogen auf das Gesamtgewicht der Zusammensetzung liegt.

11. Kosmetische oder dermatologische Zusammensetzung nach einem der vorangegangenen Ansprüche, **dadurch gekennzeichnet, dass** sie außerdem wenigstens ein Schutzmittel für die DNA der Zellen der Haut, welches in der aus den Isoflavonen und/oder den Zinksalzen bestehenden Gruppe ausgewählt wird, umfasst.

12. Kosmetische oder dermatologische Zusammensetzung nach Anspruch 11, **dadurch gekennzeichnet, dass** das Schutzmittel der Zellen der Haut Zinkgluconat ist.

13. Kosmetische oder dermatologische Zusammensetzung nach Anspruch 11 oder 12, **dadurch gekennzeichnet, dass** der Anteil des Schutzmittels der Zellen der Haut zwischen etwa 0,01 und etwa 1 Gew.-% eingeschlossen bezogen auf das Gesamtgewicht der Zusammensetzung liegt.

14. Kosmetische oder dermatologische Zusammensetzung nach einem der vorangegangenen Ansprüche, **dadurch gekennzeichnet, dass** sie außerdem wenigstens einen Hilfsstoff umfasst, der in der Gruppe, welche aus den ionischen oder nichtionischen Verdickungsmitteln, den Linderungsmitteln, den Antioxidationsmitteln, den Trübungsmitteln, den Stabilisatoren, den Weichmachern, den Insektenabwehrmitteln, den hydratisierenden Mitteln, den Vitaminen, den Parfumstoffen, den Konservierungsmitteln, den Füllstoffen, den Maskierungsmitteln, den Färbemitteln und den Mischungen von diesen Letzteren besteht, ausgewählt wird.

15. Gesamtheit für das Auftragen einer kosmetischen oder dermatologischen Zusammensetzung für den Schutz gegen die ultravioletten Strahlen, welche eine solche Zusammensetzung und ein aufnehmendes Element für diese Zusammensetzung umfasst, wobei das aufnehmende Element gebildet wird aus einem Reservoir und einer manuell betriebenen Vortriebspumpe (10, 20, 30), **dadurch gekennzeichnet, dass** die Zusammensetzung in Form einer Wasser-in-Öl-Emulsion vorliegt und dass sie wenigstens einen Emulgator, ausgewählt in der Gruppe bestehend aus den Siliconderivaten mit Glucose-Komponente, umfassend eine Anzahl von Glucose-Motiven zwischen 2 und 10 eingeschlossen, enthält, wobei das teilchenförmige anorganische Sonnenschutzmittel auf homogene Weise in der Wasser-in-Öl-Emulsion dispergiert ist und seine mittlere Teilchengröße zwischen 1 und 100 Nanometern eingeschlossen beträgt und wobei das teilchenförmige anorganische Sonnenschutzmittel in einer Konzentration von 4 bis 40 Gew.-% vorhanden ist.

16. Gesamtheit nach Anspruch 15, **dadurch gekennzeichnet, dass** die Pumpe eine Pumpe mit einer Zwischenkammer (16) mit einem gleitenden Element (40) ist, welche in der Lage ist, das innere Volumen der Zwischenkammer (10) zu fluten, um die in dieser Zwischenkammer (10) vorhandene Zusammensetzung auszustoßen.

17. Gesamtheit nach Anspruch 15 oder Anspruch 16, **dadurch gekennzeichnet, dass** die Pumpe (10, 20, 30) eine Pumpe mit einer Zwischenkammer (10) ist, die von zwei Rücklaufsicherungen (20, 30), die in der Richtung des Ausgangs des Reservoirs orientiert sind, eingerahmt wird, und dass eine der Rücklaufsicherungen (20, 30), die sich angrenzend an das Reservoir befindet, eine Versperrungskugel (20) ist.

18. Gesamtheit nach einem der Ansprüche 15 bis 17, **dadurch gekennzeichnet, dass** die Pumpe (10, 20, 30) mit einer Austrittsdüse (50) ausgestattet ist, deren Raum für die Zirkulation der Zusammensetzung einen Durchmesser zwischen 4 und 5 mm eingeschlossen aufweist.

19. Gesamtheit nach einem der Ansprüche 15 bis 18, **dadurch gekennzeichnet, dass** die Pumpe (10, 20, 30) eine Zwischenkammer (10) mit einem Volumen zwischen 100 und 200 µl eingeschlossen aufweist.

20. Gesamtheit nach einem der Ansprüche 15 bis 19, **dadurch gekennzeichnet, dass** die Pumpe ein oder mehrere Rückstellungselemente, welche das Befüllen des Zwischenvolumens sicherstellen, welche insgesamt eine Kraft über 35 N erzeugen, aufweist.

21. Gesamtheit nach einem der Ansprüche 15 bis 20, **dadurch gekennzeichnet, dass** die Pumpe eine Zwischenkammer vom zylindrischen Typ, deren Durchmesser etwa 8 mm beträgt, aufweist.

22. Gesamtheit nach einem der Ansprüche 15 bis 21, **dadurch gekennzeichnet, dass** die Pumpe mit einen Tauchrohr, dessen Innendurchmesser etwa 3,7 mm beträgt, ausgestattet ist.

23. Verwendung der Zusammensetzungen nach einem der Ansprüche 1 bis 14 für die Herstellung von dermatologischen Zusammensetzungen, die dazu bestimmt sind, durch eine Flasche mit Vortriebspumpe auf der Haut verteilt zu werden mit dem Ziel, die Haut gegen die Schädlichkeit und die Aggression der ultravioletten Strahlen zu schützen.

24. Nicht-therapeutisches Verfahren zur kosmetischen Behandlung der Haut, welches dazu bestimmt ist, die Haut vor der Schädlichkeit und der Aggression der ultravioletten Strahlen zu schützen, und welches darin besteht, durch eine Flasche mit Vortriebspumpe eine wirksame Menge der Zusammensetzung mit Hilfe einer Gesamtheit nach einem der Ansprüche 15 bis 22 zu verteilen.

25. Verwendung einer Vorrichtung vom Typ Flasche mit Vortriebspumpe für das Auftragen einer Zusammensetzung nach einem der Ansprüche 1 bis 14 auf die Haut.
